# EUROPEAN PATENT APPLICATION

(11) **EP 4 209 578 A1**
(43) Date of publication of application: **12.07.2023**
(21) Application number: 21864359.1
(22) Date of filing: 01.09.2021
(51) Int. Cl.: C12N 1/20, A23L 31/00, A23L 33/135, A61K 35/745, A61P 37/02

(54) **BIFIDOBACTERIUM INHIBITING IL-17 PRODUCTION**

(30) Priority: 02.09.2020 JP 2020147208
(71) Applicant: NISSIN FOODS HOLDINGS CO., LTD., Yodogawa-ku Osaka-shi, Osaka 532-8524 (JP)
(72) Inventor: TANII, Yusuke, Osaka-shi, Osaka 532-8524 (JP); SUNADA, Yosuke, Osaka-shi, Osaka 532-8524 (JP); UEHARA, Kazuya, Osaka-shi, Osaka 532-8524 (JP); IGI, Akemi, Osaka-shi, Osaka 532-8524 (JP); MORI, Ayaka, Osaka-shi, Osaka 532-8524 (JP)
(74) Representative: Mewburn Ellis LLP
(86) International application number: PCT/JP2021/032090
(87) International publication number: WO 2022/050302

(57) **Abstract**

The present invention provides a bifidobacterium derived from infant intestines that inhibit the production of IL-17 and can be suitably used in foods and beverages, and food and beverages containing the Bifidobacterium. Provided is *Bifidobacterium longum* species that inhibit the production of IL-17. The bifidobacterium is *Bifidobacterium longum* strain N714 (NITE BP-03004). This may reduce chronic inflammatory diseases caused by enhanced IL-17.

## Description

### TECHNICAL FIELD

The present invention relates to a strain of a bifidobacterium derived from the intestine of an infant which strain suppresses the production of IL-17, and food and beverage products containing the strain. In particular, the present invention relates to *Bifidobacterium longum.*

### BACKGROUND ART

Known cells involved in immunity include Type 1 helper T (Th1) cells, which are involved in cellular immunity, and Th2 cells, which are involved in humoral immunity. A new subset of T cells called Th17 cells has also been discovered in recent studies, besides Th1 and Th2 cells. These Th17 cells have been found to play a central role in allergic responses, autoimmunity, and some infection defenses. Th17 cells are named for their characteristic production of interleukin-17 (IL-17).

Interleukin (IL) is a general term for bioactive substances produced by immune cells, such as lymphocytes, monocytes, and macrophages, and is a proteinaceous substance that mediates intercellular interactions related to immune responses. Among these, IL-17 is mainly produced by Th17 cells and is known to induce various factors, such as inflammatory cytokines, chemokines, and cell adhesion factors, by acting on cells, such as fibroblasts, epithelial cells, vascular endothelial cells, and macrophages, to cause inflammation (See NPLs 1 and 2).

IL-17 contains several subsets, and Th17 cells produce IL-17A and IL-17F. IL-17A and IL-17F have 50% homology at the amino acid level and the corresponding receptors are identical, suggesting that many functions overlap. It is also known that human Th17 cells produce more IL-17F than IL-17A.

The enhancement in IL-17 has also been found to be associated with several chronic inflammatory diseases, including rheumatoid arthritis, psoriasis, and multiple sclerosis (see NPLs 3 and 4). Therapies targeting IL-17 are accordingly being explored for the treatment of chronic inflammatory diseases.

### CITATION LIST

### NONPATENT LITERATURE

[NPL 1] Aggarwal S, Gurney AL. IL-17: prototype member of an emerging cytokine family. J Leukoc Biol 2002; 71: 1-8.
[NPL 2] Kolls JK, Linden A. Interleukin-17 family members and inflammation. Immunity 2004; 21: 467-76.
[NPL 3] Nakae S, NambuA, Sudo K, et al. Suppression of immune induction of collagen-induced arthritis in IL-17-deficient mice. J Immunol 2003; 171: 6173-7.
[NPL 4] Komiyama Y, Nakae S, Matsuki T, et al. IL-17 plays an important role in the development of experimental autoimmune encephalomyelitis. J Immunol 2006; 177: 566-73.

### SUMMARY OF INVENTION

### TECHNICAL PROBLEM

In this study, the inventors have investigated whether the production of IL-17 could be suppressed by diet and have found that bifidobacteria isolated from infant feces contain a bifidobacterium that inhibits the production of IL-17, leading to the completion of the present invention.

### SOLUTION TO PROBLEM

To solve the above problem, the present invention is characterized by a bifidobacterium belonging to the genus Bifidobacterium that inhibit the production of IL-17.

To solve the above problem, the present invention is also characterized in that the bifidobacterium is *Bifidobacterium longum* strain N714 (NITE BP-03004).

To solve the above problem, the present invention is further characterized by a food and beverage containing the bifidobacterium

### ADVANTAGEOUS EFFECTS OF INVENTION

The bifidobacteria of the present invention inhibit the production of IL-17. This can thereby reduce chronic inflammatory diseases caused by increased IL-17.

### BRIEF DESCRIPTION OF DRAWINGS

Fig. 1 is a graph that compares the inhibitory effect on IL-17F production of the inventive and comparative strains.
Fig. 2 is a graph that compares the inhibitory effect on IL-17F production between the strains of the present invention and the reference strain.

### DESCRIPTION OF EMBODIMENTS

The invention will now be described in detail.

### 1. Bifidobacterium longum strain N714 (NITE BP-03004)

The bifidobacterium in the present invention is *Bifidobacterium longum.* The symbol N714 in the present invention is a number independently assigned to the strain by Nissin Foods Holdings Co. Ltd. The strain was first isolated by the inventor from infant feces.

The *Bifidobacterium longum* strain N714 is deposited under the following conditions:
(1) Name of depositary: Patent Microorganisms Depositary Center of National Institute of Technology and Evaluation (NITE)
(2) Contact: Room 122, 2-5-8 Kazusa-Kamatari, Kisarazu, Chiba 292-0818, Japan
(3) Trustee number: NITE BP-03004
(4) Indication for identification: N714
(5) Consignment date: July 10, 2019

The bacteriological characteristics of *Bifidobacterium longum* strain N714 are shown in Tables 1 and 2 below. The bacteriological characteristics are determined based on the method described in Bergey's manual of systematic bacteriology Vol. 2 (1986). Table 1 shows the shape and other characteristics related to this strain, and Table 2 shows the results of physiological and biochemical characterizations using Api 50CH and Api CHL (Biomérieux). In Tables 1 and 2, the symbol "+" indicates positive and the symbol "-" indicates negative.

**[Table 1]**

| | | |
|---|---|---|
| Cultivation temperature (°C) | | 37°C |
| Shape of cells | | Bacillus |
| | | 0.5 × 2.0-4.0 µm |
| Gram stain | | + |
| Spores | | - (Not found) |
| Motility | | - |
| Colony form | Culture medium | GAM agar medium |
| | Incubation time | 24 hours |
| | Diameter | 0.5-2.0 mm |
| | Color | Cream |
| | Shape | Circular |
| | Apophysis | Lenticular |
| | Fringe | Entire margin |
| | Surface | Smooth |
| | Transparency | Translucent |
| | Viscosity | Buttery |
| Growth temperature test | 30°C | + |
| | 45°C | - |
| Catalase reaction | | - |
| Oxidase reaction | | - |
| Acid/gas generation from glucose (acid acidic/gas acidic) | | +/- |
| O/F test (oxidation/fermentation) | | +/+ |

| | | |
|---|---|---|
| +: positive, -: negative | | |

**[Table 2]**

| | | |
|---|---|---|
| 0 | Control | - |
| 1 | Glycerol* | - |
| 2 | Erythritol* | - |
| 3 | D-Arabinose* | - |
| 4 | L-Arabinose* | + |
| 5 | Ribose | + |
| 6 | D-Xylose* | + |
| 7 | L-Xylose* | - |
| 8 | Adonitol* | - |
| 9 | Beta-Methyl-D-Xylose* | - |
| 10 | Galactose* | + |
| 11 | Glucose*. | + |
| 12 | Fructose* | + |
| 13 | Mannose* | + |
| 14 | Sorbose* | - |
| 15 | Rhamnose* | - |
| 16 | Zulcitol*. | - |
| 17 | Inositol*. | - |
| 18 | Mannitol* | + |
| 19 | Sorbitol* | + |
| 20 | Alpha-methyl-D-mannoside* | - |
| 21 | α-Methyl-D-glucoside^{∗} | - |
| 22 | N-acetylglucosamine* | - |
| 23 | Amygdalin* | - |
| 24 | Arbutin* | - |
| 25 | Esculin*. | + |
| 26 | Salicin* | - |
| 27 | Cellobiose* | - |
| 28 | Maltose* | + |
| 29 | Lactose* | + |
| 30 | Melibiose* | + |
| 31 | Sucrose* | + |
| 32 | Trehalose* | - |
| 33 | Inulin* | - |
| 34 | Meletitose* | - |
| 35 | Raffinose* | + |
| 36 | Starch* | - |
| 37 | Glycogen* | - |
| 38 | Xylitol* | - |
| 39 | Gentiobiose* | - |
| 40 | D-Turanose* | + |
| 41 | D-Liquisose* | - |
| 42 | D-Tagatose* | - |
| 43 | D-Fucose* | - |
| 44 | L-Fucose* | - |
| 45 | D-Arabitol* | - |
| 46 | L-Arabitol* | - |
| 47 | Gluconate* | - |
| 48 | 2-Ketogluconate* | - |
| 49 | 5-Ketogluconate* | - |

| | | |
|---|---|---|
| *Fermentability test +: Positive -: Negative | | |

### 2. Inhibitory test of IL-17 production

The *Bifidobacterium longum* strain N714 of the present invention has a high ability to inhibit IL-17 production, as shown in the experimental examples described below. The inhibition of IL-17 production was confirmed by the following method.

### <Preparation of samples used in inhibitory test of IL-17 production>

Samples used in the inhibitory test of IL-17 production were prepared by culturing bifidobacteria in a GAM medium (Nippon Suisan Kaisha, Ltd.) shown in Table 3 at 37°C for 24 hours. The proliferated bacteria were then collected by centrifugation. The collected bacteria were washed with sterile water and collected with a centrifuge. Washing and collection were repeated three times. Heat sterilization was then applied, and the heat sterilized samples were freeze-dried with a freeze-dryer to yield dried bacterial powder.

**Table 3**

| | |
|---|---|
| Peptone | 10.00 g |
| Soybean peptone | 3.00 g |
| Proteose peptone | 10.00 g |
| Digestive Serum powder | 13.50 g |
| Yeast extract | 5.00 g |
| Meat extract | 2.20 g |
| Liver extract | 1.20 g |
| Glucose | 3.00 g |
| Potassium dihydrogen phosphate | 2.50 g |
| Sodium chloride | 3.05 g |
| Soluble starch | 5.00 g |
| L-Cysteine hydrochloride | 0.30 g |
| Sodium thioglycolate | 0.30 g |
| Distilled water | 1000 mL |

### <Inhibitory test of IL-17 production>

Human peripheral blood mononuclear cells (hPBMC, CTL) were used in the inhibitory test of the IL-17 production. Using an IMDM medium (Sigma) supplemented with 10% fetal bovine serum and 4 mM GlutaMAX (Gibco), cells were seeded into 1.0 × 10⁶ cells/well and the sample prepared above was seeded into 10 ng/well on a 96-well plate. As Th17 cell differentiation stimulants, 25 ng/mL rhIL-6 (BioLegend), 12.5 ng/mL rhIL-23 (BioLegend), 25 ng/mL rhIL-21 (BioLegend), 2 ng/mL rhTGF-β1 (BioLegend), and 25 ng/mL rhIL-1β (BioLegend) were added for incubation for 96 hours at 37°C in 5% CO₂. After incubation, the culture supernatant of hPBMC was collected and IL-17F in the supernatant was measured with a commercially available immunoassay kit (R&D).

### 3. Food and beverage products

The bifidobacterium of the present invention can be used by being compounded in food and beverages. The bifidobacterium of the present invention is particularly suitable for use in beverages, for example, fermented milk and lactic acid bacteria beverages. According to the current Ministerial Ordinance on Standard of Ingredients for Milk and Dairy Products, the ingredient standards require a minimum of 1.0 × 10⁷ cfu/mL for fermented milk (with a solid content of nonfat milk of 8.0% or more) and dairy lactic acid bacteria beverages (with a solid content of nonfat milk of 3.0% or more) and a minimum of 1.0 × 10⁶ cfu/mL for lactic acid bacteria beverages (with a solid content of nonfat milk less than 3.0%), but the above bacteria counts can be achieved by growing in fermentation solutions such as milk or in the form of the final product. The bifidobacterium can also be used in dairy products such as butter, processed egg products such as mayonnaise, and pastries such as butter cake, in addition to fermented milk and lactic acid bacteria beverages containing the bifidobacterium. The bifidobacteria can also be suitably used in processed foods, such as instant noodles and cookies. In addition to these applications, the food products of the present invention may be in formulated forms (e.g., powder, granules, capsules, and tablets) of the bifidobacteria together with appropriate carriers and additives as necessary.

The bifidobacterium of the present invention is also useful to be compounded in foods for specified health uses and nutritional supplements in addition to general beverages and foods. The bifidobacterium of the present invention can also be applied in cosmetics fields, such as lotion, pharmaceuticals fields, such as intestinal regulators, daily necessities fields, such as toothpaste, and animal feed and plant fertilizer fields, such as silage, animal feed, and plant liquid fertilizer, other than foods.

### INDUSTRIAL APPLICABILITY

The bifidobacterium (*Bifidobacterium longum* strain N714) is highly active in inhibiting the production of IL-17.

### [Examples]

Examples of the present invention will now be described, but the present invention is not limited to the following examples.

### <Example 1> Inhibitory test of IL-17 production

*Bifidobacterium longum* strain N714 of the present invention and *Bifidobacterium longum* comparison strain owned by the company were subjected to inhibitory test of IL-17 production.

Each of the strain of the present invention, the reference strain described below, and the comparative strains was incubated at 37°C for 24 hours in the GAM medium (Nippon Suisan Kaisha, Ltd.) shown in Table 3. AnaeroPac (Mitsubishi Gas Chemical Company, Inc.) was used to culture the bacteria under anaerobic conditions. The bacteria were then collected by centrifugation. The collected bacteria were washed three times with sterile water, heat sterilized, and frozen. The bacteria were then freeze-dried with a freeze-dryer to yield dried bacteria powder. The resulting dried bacterial powder was suspended in PBS (-) as bifidobacterial suspension.

hPBMC was seeded in 96-well plates at 1.0 × 10⁶ cells/well. Each sample group was then added into a final concentration of 10 ng/mL. Th17 cell differentiation stimulants of 25 ng/mL rhIL-6 (BioLegend), 12.5 ng/mL rhIL-23 (BioLegend), 25 ng/mL rhIL-21 (BioLegend), 2 ng/mL rhTGF-β1 (BioLegend), and 25 ng/mL rhIL-1β (BioLegend) were added for incubation for 96 hours at 37°C in 5% CO₂. After incubation, the culture supernatant of hPBMC was collected and IL-17 in the supernatant was measured with a commercially available immunoassay kit (R&D). Samples not containing both bifidobacterial suspension and Th17 cell differentiation stimulants was defined as "not stimulated", whereas samples containing only Th17 cell differentiation stimulants was defined as "stimulated".

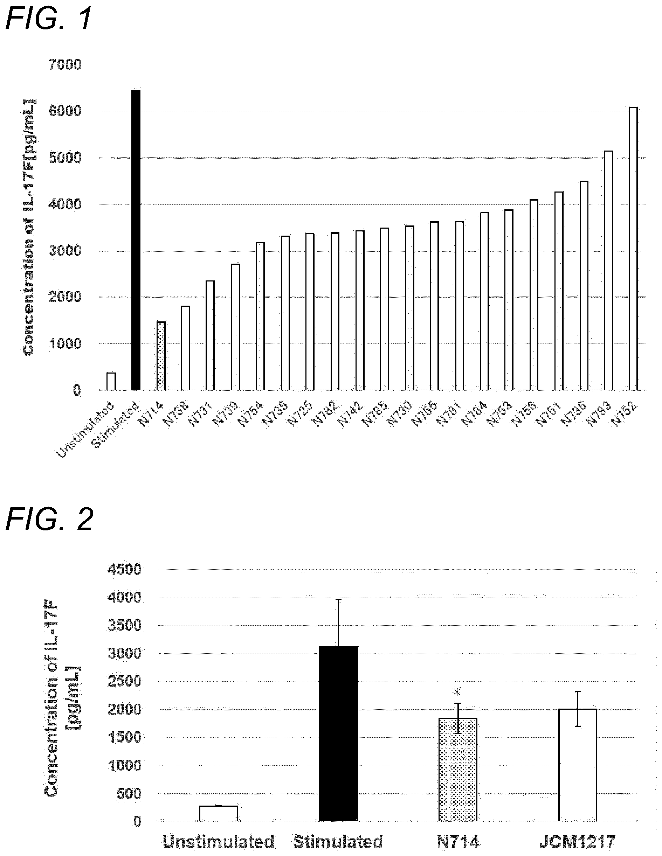

Fig. 1 demonstrates the concentration (pg/mL) of IL-17F in the culture supernatant in the case of addition of each sample group.

Fig. 1 demonstrates that some bifidobacteria inhibit IL-17F production, while others do not. The strain N714 has the highest activity for inhibition of IL-17F production in comparison with the other bifidobacteria, although the production of IL-17F was higher than unstimulated sample. The low concentration of unstimulated IL-17F may be due to the failure to differentiate into Th17 cells. Although we have no certainty as to the cause of the suppression of IL-17F production in strain N714, we speculate at the present stage as follows: One possibility is that the addition of the strain N714 suppressed Th17 cell differentiation itself, resulting in suppression of IL-17F production. Another possibility is that IL-17 production is suppressed for some reason although Th17 cells are differentiated. Either or both of these reasons, or some other reason, may be responsible for the suppression of IL-17 production.

IL-17F was used as an indicator to evaluate IL-17 in this study because IL-17F has the highest production among the IL-17 subsets and is easy to detect. However, the amount produced of subsets other than IL-17F is very low and difficult to detect. Thus, IL-17F was used as the index for evaluation in this study. It is assumed that the production of subsets other than IL-17F is also suppressed by *Bifidobacterium longum* N714, based on the above-mentioned inference.

### <Example 2> Evaluation of the superiority of Bifidobacterium longum strain N714

The *Bifidobacterium longum* strain N714 was then evaluated in comparison with the typestrain, *Bifidobacterium longum* JCM1217, to confirm the superiority of the *Bifidobacterium longum* strain N714. The test was performed three times as in Example 1, and the average value was used for comparison with the stimulated group. The results are shown in Fig. 2.

Fig. 2 evidentially shows that the production of IL-17 was significantly suppressed in the group containing *Bifidobacterium longum* strain N714 in comparison with the stimulated group.

As described above, *Bifidobacterium longum* strain N714 has a function that effectively inhibits the production of IL-17.

## Claims

1. A bifidobacterium belonging to *Bifidobacterium longum* species that inhibits production of IL-17.

2. The bifidobacterium according to claim 1, wherein the bifidobacterium is *Bifidobacterium longum* strain N714 (NITE BP-03004).

3. A food or drink containing the bifidobacterium according to claim 1 or 2.
